# EUROPEAN PATENT APPLICATION

(11) **EP 0 718 404 A2**
(43) Date of publication of application: **26.06.1996**
(21) Application number: 95118252.6
(22) Date of filing: 20.11.1995
(51) Int. Cl.: C12N 15/63

(54) **Method for site-directed mutagenesis**

(30) Priority: 22.11.1994 JP 311306/94
(71) Applicant: TAKARA SHUZO CO. LTD., Fushimi-ku, Kyoto-shi, Kyoto-fu (JP)
(72) Inventor: Yamashita, Hiroshige, Kusatsu-shi, Shiga (JP); Tomono, Jun, Otsu-shi, Shiga (JP); Kita, Akihiko, Otsu-shi, Shiga (JP); Noda, Akihiro, Gamo-gun, Shiga (JP); Nakajima, Kazuo, Kyoto-shi, Kyoto (JP); Kato, Ikunoshin, Uji-shi, Kyoto (JP)
(74) Representative: Weisert, Annekäte, Dipl.-Ing. Dr.-Ing.

(57) **Abstract**

The present invention provides a simplified method for site-directed mutagenesis and a kit to be used therefor.
The method for site-directed mutagenesis comprises the steps of:
(1) treating a vector containing the target DNA for the site-directed mutagenesis and a DNA for the mutagenesis with a protein capable of catalyzing a strand exchange reaction *in vitro* to thereby prepare a vector having a mutation introduced thereinto via the strand exchange reaction; and (2) introducing the resulting vector, which has the mutation introduced thereinto, into host cells. A kit for site-directed mutagenesis comprises a protein capable of catalyzing a strand exchange reaction.

## Description

### FIELD OF THE INVENTION

This invention relates to a simplified method for site-directed mutagenesis employed in the field of genetic engineering and a kit to be used therefor.

### BACKGROUND OF THE INVENTION

In recent years, site-directed mutagenesis has become an indispensable technique for clarifying a relationship between the structure and function of a gene cloned in the field of genetic engineering. In the field of protein engineering, site-directed mutagenesis is also an indispensable technique for modifying a protein to thereby alter its properties.

Site-directed mutagenesis is conventionally performed, for example, by the following procedures.
(1) A DNA, to which the aimed mutation is to be introduced, is inserted into a vector. In the case of a double-stranded plasmid DNA, the complementary strand is then dissociated by thermal denaturation or, alternatively, an M13 phage vector is used to thereby give a single-stranded DNA.
(2) An oligonucleotide, which has been chemically synthesized in accordance with the aimed mutation, is annealed with the above-mentioned single-stranded DNA and then subjected to a polymerase reaction and a ligase reaction to thereby give a double-stranded DNA which is complementary except the aimed mutation.
(3) The above-mentioned DNA is used to transform *Escherichia coli* and a clone having a mutation introduced thereinto is selected.

This site-directed mutagenesis method requires complicated and troublesome procedures.

On the other hand, there have been known several proteins which are capable of catalyzing strand exchange reactions. Among these proteins, detailed studies have been made on the properties of one called RecA which originates in *Escherichia coli* and attempts have been made to use this RecA protein in mutagenesis [Tanpakushitsu, Kakusan, Koso (Protein, Nucleic Acid and Enzyme), 32(1), 73 - 74 (1987)]. In this method, however, a mutation is introduced not directly via a strand exchange reaction but randomly via treatments with mutagenic agents. Thus the mutation can be hardly introduced into a definite site thereby and thus this method cannot be regarded as site-directed mutagenesis.

### SUMMARY OF THE INVENTION

An object of the present invention is to provide a simplified method for site-directed mutagenesis and a kit for the embodiment of this method.

The first aspect of the present invention relates to a method for site-directed mutagenesis comprising the steps of:
(1) treating a vector containing the target DNA for the site-directed mutagenesis, and a DNA for the mutagenesis with a protein capable of catalyzing a strand exchange reaction *in vitro* to thereby prepare a vector having a mutation introduced thereinto via the strand exchange reaction; and
(2) introducing the vector, which has the mutation introduced thereinto, into host cells.

The second aspect of the present invention relates to a kit for site-directed mutagenesis comprising a protein capable of catalyzing a strand exchange reaction.

### DETAILED DESCRIPTION OF THE INVENTION

The present invention will be described in detail below.

The site-directed mutagenesis according to the method of the present invention can be carried out, for example, in the following manner.
(1) A vector, to which the target DNA for the site-directed mutagenesis has been inserted, and a DNA for the mutagenesis are treated with a protein capable of catalyzing a strand exchange reaction *in vitro* to thereby induce homologous recombination.
(2) The vector prepared in the above (1) is introduced into host cells. In this step, it is preferable to apply selective pressure so as to avoid the selection of a vector having no mutation introduced therein.
(3) The DNA in which the mutation has been introduced is obtained by extracting the vector. If necessary, the extracted vector is introduced into an appropriate host again to thereby obtain a DNA into which the mutation has been introduced without fail.

The DNA to be used as a target for the mutagenesis is not particularly limited as long as it does not exert lethal activity in the host cells. When the vector is double-stranded, the DNA for the mutagenesis (single-stranded) and the vector is used in a molar ratio of about 1 : 2.

RecA protein originating in *Escherichia coli* may be cited as a typical example of the protein capable of catalyzing a strand exchange reaction. However, the present invention is not restricted thereto. Namely, proteins being similar to RecA but originating in other sources and variants thereof are usable herein. The proteins with other sources are exemplified by a Rad51 gene product originating in a fission yeast. There have been cloned similar genes in mouse and human being and products of these genes are also usable. When a mutation is to be introduced using RecA protein, the reaction may be performed in a buffer such as Tris-HCl buffer or HEPES containing a magnesium salt, such as magnesium acetate or magnesium chloride, and adenosine triphosphate (ATP). Preferable reaction medium has, for example, the composition containing 25 mM Tris-HCl (or Tris acetate) (pH 7.5), 10 mM MgCl₂, 1 mM DTT and 1 mM ATP or the composition containing 30 mM HEPES (pH 7.2), 15 mM Mg acetate, 2 mM DTT, 1.2 mM ATP and 100 µg/ml BSA. In this case, the RecA protein may be used in a concentration effective to allow the strand exchange reaction to proceed. For example, when the vector is double-stranded, the vector and the protein can be used in an concentration of about 2.5 to 20 µM and about 5 to 40 µM, respectively.

The reaction medium may further contain ATP reproducing system, for example, containing 8 mM phosphocreatine and 10 U/ml creatine phosphokinase. In this case, the amount of the protein may be reduced to about 2 µM.

The strand exchange reaction can be carried out by mixing the components other than the protein, prewarming the mixture, adding the protein thereto and incubating the reaction mixture at 37 °C for 1 to 2 hours.

Examples of the host cells to be used in the step (2) include cells with an established host-vector system, i.e., bacteria such as *Escherichia coli* and *Bacillus subtilis*, yeasts, fungi, plant cells and animal cells. It is preferable to use *Escherichia coli* therefor from the viewpoint of convenience in handling. Although any strain of *Escherichia coli* may be used without restriction, it is more preferable to use a strain having an mutS mutation such as BMH71-18mutS strain. Other mismatch-repairing system-deficient strains such as mutT or mutD may be used.

The vector to be used in the step (1) may be either a single-stranded vector or a double-stranded one. An appropriate vector may be selected therefor depending on the host cells employed. When *Escherichia coli* is employed as the host cells, examples of the vectors to be used include pUC-series, pBR-series or M13.

The DNA for the mutagenesis is a natural or synthetic DNA containing the aimed mutation. For example, a single-stranded synthetic oligonucleotide may be used. It is needed that this oligonucleotide has such a length as to allow stable hybridization with the target sequence, in general, 20 mer or longer.

In the case of intermolecular homologous recombination, the longer homologous sequences ensure the occurrence of the recombination. The site-directed mutagenesis can be carried out more efficiently by using a long chain DNA such as a PCR product or the like as the DNA for mutagenesis. For example, PCR is effected using, as a pair of primers, an oligonucleotide, which contains the aimed mutation, and another oligonucleotide, which is complementary to the site of the vector other than the region to which the mutation is to be introduced. Then, the resulting PCR product is used as the DNA for mutagenesis. Thus a long chain DNA, which can be hardly made by chemical synthesis can be easily prepared. The use of a longer chain DNA makes it possible to provide the more stable efficiency. The length of the DNA for the mutagenesis generally ranges from several tens bp to several kbp, which falls within the size of the vector into which the DNA is introduced.

This method is particularly effective in such a case that it is difficult to establish stable hybridization by using an oligonucleotide, the aimed mutation cannot be introduced because of the existence of a site highly complementary to an oligonucleotide other than the mutagenesis site, or a host with a low transformation rate is employed.

The combination of the vector and the DNA for mutagenesis may be one capable of forming a so-called D-loop or a double D-loop [Nature Genetics, 3, 365 - 371 (1993)].

For the introduction of the DNA into the host, a method by which a high transformation rate can be achieved depending on the host can be selected. For example, electroporation may be used.

By applying selective pressure in the step (2), the aimed DNA can be selected more efficiently. The selective pressure may be applied in, for example, the following manner. When *Escherichia coli* is used as the host, the vector is linearized by cleaving at an appropriate restriction enzyme cleavage site within a region corresponding to the DNA for mutagenesis and then subjected to the strand exchange reaction. When the homologous recombination occurs due to the strand exchange reaction, the vector is cyclized again. When the strands are unreacted and thus no homologous recombination occurs, on the other hand, the vector remains linear. When *Escherichia coli* is employed as the host, the introduction rate of a linear DNA is generally lower than that of a cyclic DNA by 10⁻² to 10⁻³. Therefore, the cyclic DNA, which has the aimed mutation introduced thereinto, is preferentially introduced into the host. As a result, the aimed DNA can be selected more efficiently. This method is particularly preferable since it is applicable to any vector without restriction similar to the case of applying selective pressure through amber mutation. When the above-described selective pressure is not applied, the desired colony can be selected by color selection using β-galactosidase or green fluorescein protein (GFP).

In the method of the present invention, it is not necessary to prepare a single-stranded DNA through phage infection or denaturation as done in the conventional methods since a double-stranded vector can be employed. Moreover, it requires no reaction step catalyzed with two or more enzymes (polymerase reaction, ligase reaction, etc. ). Since a protein capable of catalyzing a strand exchange reaction is used, further, the method of the present invention makes it possible to stably introduce the mutation regardless of the base sequence of the DNA for mutagenesis, compared with the conventional method involving an annealing step.

The site-directed mutagenesis method of the present invention can be further easily carried out by assembling the reagents to be used therein into a kit. The kit contains at least a protein capable of catalyzing a strand exchange reaction. It may contain a reaction buffer. It may also contain a vector and a synthetic oligonucleotide both for control. Each component can be provided in the form of a solution.

To further illustrate the present invention in greater detail, and not by way of limitation, the following Examples will be given.

### EXAMPLE 1

### (1) Construction of pUC19-Mut

A plasmid pUC19-Mut, wherein G at the 30-position in the downstream of the polycloning site in lacZ' gene of a plasmid pUC19 had been replaced by A, was constructed as the vector to be used in the following Examples. The lacZ' gene, which encodes lacZα-peptide, exerts β-galactosidase activity when introduced into a host having a genotype of lacZΔM15. In the presence of IPTG, the lacZ' gene which undergoes no base replacement gives a blue colony due to the reaction in which X-gal serves as a substrate. On the contrary, the mutated lacZ' gene resulted from the base replacement cannot exert its inherent activity and thus gives a white colony.

By utilizing these phenomena, the reversion rate of the base replacement in the mutated lacZ' gene of pUC19-Mut by site-directed mutagenesis can be calculated by counting blue and white colonies.

### (2) Synthesis of oligonucleotide to be used as DNA for mutagenesis

As a DNA for mutagenesis aiming at the reversion of the base replacement in the lacZ' gene, a 5'-end phosphorylated oligonucleotide (dR2) represented by SEQ ID NO. 1 of the Sequence Listing was synthesized. Namely, dR2 was designed to give the activated lacZ' gene in which the base replacement was reversed by the C at the 12-position.

### (3) Strand exchange reaction catalyzed by RecA protein

To 1 µl of 10 x reaction buffer [250 mM Tris acetate (pH 7.5), 100 mM magnesium acetate, 10 mM dithiothreitol, 25 mM ATP] were added 200 pmol of RecA protein (manufactured by Pharmacia Biotech), 50 ng of pUC19-Mut and 100 pmol of dR2. After adjusting the total volume to 10 µl with sterilized water, the mixture was allowed to react at 37 °C for 1 to 2 hours.

After the completion of the reaction, Proteinase K (manufactured by Takara Shuzo, Co., Ltd.) was added thereto in such a manner as to give a concentration of 10 mg/ml to remove the RecA protein. Next, the excessive dR2 was removed by using Microcon 100 (manufactured by Takara Shuzo Co., Ltd.).

### (4) Introduction into host Escherichia coli by electroporation

Introduction into the host *Escherichia coli* (BMH71-18mutS strain) was carried out in the following manner using Gene Pulser (manufactured by Bio-Rad).

First, 100 µl of BMH-71-18mutS competent cells (manufactured by Takara Shuzo Co., Ltd.) were added to an M9 medium containing 1 mM of thiamine and incubated therein at 37 °C overnight under shaking. Five ml of this culture medium was further inoculated into 500 ml of an LB medium. When the absorbance at 600 nm reached about 0.8, the cells were harvested by centrifuging at 4,000 rpm for 8 minutes and suspended in 1 ml of a 10 % solution of glycerol.

Then, several µl of the reaction mixture prepared in the above (3) was added to 50 µl of the above-mentioned suspension. The resulting mixture was poured into a cuvette of 0.1 cm in width, which had been preliminarily cooled in ice, and then a pulse of 1.5 kV was applied thereto. Immediately thereafter, 1 ml of an SOC medium was added followed by the incubation at 37 °C for 1 hour under shaking. After the completion of the incubation, the cells were harvested, applied onto an LB agar medium containing 100 µg/ml of ampicillin and X-gal and then incubated at 37 °C overnight. Then the colonies thus growing in the medium were counted. Colonies of the lacZ' gene with the reverse mutation had a blue color, while those having no reversion had a white color.

As a result, blue colonies appeared at a ratio of 1 per about 1,000 colonies. In the control case wherein neither RecA protein nor dR2 was added, no blue colony was observed. In other words, all colonies were white. Furthermore, the mutation site was analyzed by sequencing. Consequently, a blue colony showed a band corresponding to A and another band corresponding to G thus reversed, while a white colony showed the band of A alone.

Then, the blue colony thus obtained was inoculated into 2 ml of an L-medium containing 100 µg/ml of ampicillin and incubated at 37 °C overnight. From the cells thus obtained, a plasmid was extracted. By using this plasmid, *Escherichia coli* JM109 was transformed and applied to an L-agar medium containing ampicillin and X-gal. As a result, blue colonies and white colonies appeared at a ratio of about 1 : 1. When sequencing was carried out by using a plasmid extracted from the blue colonies, no A but the reversed G was exclusively observed.

These results indicate that when the treatment with RecA protein is performed *in vitro*, homologous recombination due to strand exchange occurs even in an oligonucleotide involving a mismatch.

### EXAMPLE 2

### (1) Preparation of DNA for mutagenesis by PCR

PCR was carried out in the following manner with the use of an oligonucleotide (dR1), which contained the aimed mutation and was represented by SEQ ID No. 2 of the Sequence Listing, and another oligonucleotide (dR3), which was complementary with the site of the template other than the mutation-introduced site and represented by SEQ ID No. 3, as a pair of primers and pUC19-Mut of Example 1-(1) as a template. The reaction system comprised 10 ng of pUC19-Mut, 20 pmol portions of the primers dR1 and dR3, 200 µM portions of dATP, dGTP, dCTP and dTTP, 2.5 U of Ampli Taq DNA polymerase (manufactured by Takara Shuzo Co., Ltd.), 10 mM of Tris-HCl (pH 8.4), 50 mM of KCl, 2.5 mM of MgCl₂ and 0.02 % of gelatin (total volume: 100 µl). After layering mineral oil, the reaction was performed for 30 cycles with each cycle consisting of 30 seconds at 94 °C, 30 seconds at 60 °C and 1 minute at 72 °C.

After the completion of the reaction, the mineral oil which had been layered was removed. Then a 10 µl portion of the reaction mixture was taken up and electrophoresed to thereby confirm that a DNA fragment of the aimed size had been amplified. Then, the reaction mixture was concentrated to about 10 µl and purified by using Microcon 100.

### (2) Preparation of vector

The pUC19-Mut prepared in the above Example 1-(1) was linearized by digesting with a restriction enzyme BamHI. After the completion of the digestion, the vector was extracted with phenol, precipitated with ethanol and then dissolved in an appropriate amount of TE buffer.

### (3) Strand exchange reaction catalyzed by RecA protein and introduction into host Escherichia coli

The procedures of the above Example 1-(3) and (4) were repeated but replacing the pUC19-Mut and dR2 respectively with the linearized DNA prepared in Example 2-(2) and the PCR product obtained in Example 2-(1) to thereby transform BMH71-18mutS strain. As a result, blue colonies were formed at a rate of about 50 %. Then the plasmid was extracted from these blue colonies and *Escherichia coli* JM109 was transformed in the same manner as described in Example 1-(4). From the blue colonies thus formed, a DNA having the aimed mutation introduced thereinto was obtained.

### EXAMPLE 3

A kit for site-directed mutagenesis (for 50 operations) was assembled from Rec A protein, a reaction buffer, a control vector and an oligonucleotide (Table 1).

**TABLE 1**

| Component | Content |
|---|---|
| RecA protein solution (7.4 mg/ml) | 50 µl |
| 10 x reaction buffer | 50 µl |
| pUC19-Mut solution (10 ng/µl) | 10 µl |
| dR1 solution (100 pmol/µl) | 10 µl |

While the invention has been described in detail and with reference to specific examples thereof, it will be apparent to one skilled in the art that various changes and modifications can be made therein without departing from the spirit and scope thereof.

## Claims

1. A method of site-directed mutagenesis comprising the steps of:
(1) treating a vector containing a target DNA for site-directed mutagenesis and a DNA containing a desired mutation to be introduced into the target DNA, with a protein capable of catalyzing a strand exchange reaction *in vitro* to allow the strand exchange reaction to proceed; and
(2) introducing the resulting vector into host cells.

2. The method of site-directed mutagenesis according to Claim 1, wherein said protein capable of catalyzing a strand exchange reaction is RecA protein.

3. The method of site-directed mutagenesis according to Claim 1, wherein said host cells are selected from the group consisting of bacteria, yeasts, fungi, plant cells and animal cells.

4. The method of site-directed mutagenesis according to Claim 1, wherein said vector is selected from the group consisting of pUC-series, pBR-series and M13 and the host is *Escherichia coli*.

5. The method of site-directed mutagenesis according to Claim 4, wherein said vector is cleaved with a restriction enzyme at a site within a region corresponding to the DNA complementary to the target DNA before the strand exchange reaction.

6. The method of site-directed mutagenesis according to Claim 1, which further comprises the step of (3) recovering the vector in which the desired mutation has been introduced from the host cells.

7. A kit for site-directed mutagenesis comprising a protein capable of catalyzing a strand exchange reaction.

8. The kit for site-directed mutagenesis according to Claim 7, which further comprises a buffer for the strand exchange reaction.

9. The kit for site-directed mutagenesis according to Claim 7, which further comprises a vector and a synthetic oligonucleotide both for control.

10. The kit for site-directed mutagenesis according to Claim 7, wherein said protein capable of catalyzing a strand exchange reaction is RecA protein.


SEQUENCE LISTING

(1) GENERAL INFORMATION:

(i) APPLICANT:
(A) NAME: TAKARA SHUZO CO., LTD.
(B) STREET: 609, Takenaka-cho, Fushimi-ku
(C) CITY: Kyoto-shi
(D) STATE: Kyoto
(E) COUNTRY: JAPAN
(F) POSTAL CODE (ZIP):

(ii) TITLE OF INVENTION: METHOD FOR SITE-DIRECTED MUTAGENESIS

(iii) NUMBER OF SEQUENCES: 3

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(vi) PRIOR APPLICATION DATA:
(A) APPLICATION NUMBER: JP HEI-6-311306
(B) FILING DATE: 22-NOV-1994

(2) INFORMATION FOR SEQ ID NO: 1:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 1:

CAGGGTTTTC CCAGTCACGA CG 22

(2) INFORMATION FOR SEQ ID NO: 2:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 2:

GGGTAACGCC AGGGTTTTCC CAGTCACGAC G 31

(2) INFORMATION FOR SEQ ID NO: 3:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(xi) SEQUENCE DESCRIPTION: SEQ ID NO: 3:

GAGCGGATAA CAATTTCACA CAGG 24